# EUROPEAN PATENT SPECIFICATION

(11) **EP 3 094 334 B1**
(45) Date of publication and mention of the grant of the patent: **14.10.2020**
(21) Application number: 15710423.3
(22) Date of filing: 13.01.2015
(51) Int. Cl.: A61K 31/593, A61K 31/663, A61K 31/675

(54) **PHARMACEUTICAL COMPOSITION COMPRISING A COMBINATION OF A BISPHOSPHONATE AND CHOLECALCIFEROL AND METHOD FOR THE PREPARATION THEREOF**
PHARMAZEUTISCHE ZUSAMMENSETZUNG, ENTHALTEND EINE KOMBINATION AUS EINEM BISPHOSPHONAT UND CHOLECALCIFEROL UND VERFAHREN ZU DEREN HERSTELLUNG
COMPOSITION PHARMACEUTIQUE RENFERMANT UNE COMBINAISON D'UN BISPHOSPHONATE ET DU CHOLÉCALCIFÉROL ET SON PROCÉDÉ DE PRÉPARATION

(30) Priority: 14.01.2014 GR 20140100029
(43) Date of publication of application: 23.11.2016
(73) Proprietor: Pharmathen S.A., 15351 Pallini Attikis (GR)
(72) Inventor: KARAVAS, Evangelos, GR-15351 Pallini Attikis (GR); KOUTRIS, Efthymios, GR-15351 Pallini Attikis (GR); SAMARA, Vasiliki, GR-15351 Pallini Attikis (GR); KOUTRI, Ioanna, GR-15351 Pallini Attikis (GR); KIZIRIDI, Christina, GR-15351 Pallini Attikis (GR); ABATZIS, Morfis, GR-15351 Pallini Attikis (GR); KALASKANI, Anastasia, GR-15351 Pallini Attikis (GR); GIANNAKOPOULOS, Spilios, GR-15351 Pallini Attikis (GR)
(86) International application number: PCT/EP2015/000037
(87) International publication number: WO 2015/106960

(56) References cited:
- EP-A1- 2 201 937
- US-A1- 2010 048 511
- US-A1- 2010 179 110

## Description

### TECHNICAL FIELD OF THE INVENTION

The present invention relates to a stable pharmaceutical composition for oral administration comprising in a single dosage form, a therapeutically effective quantity of a bisphosphonate such as Alendronate or pharmaceutical acceptable salt, derivative or hydrate thereof and Cholecalciferol and a method for the preparation thereof.

### BACKGROUND OF THE INVENTION

A variety of disorders in humans are associated with abnormal bone resorption. Among the most common of these disorders is osteoporosis, which is a systemic skeletal disease leading to bone fragility and susceptibility to fracture. More specifically, postmenopausal osteoporosis is characterized by a reduction in bone mass and strength resulting from a compromise of bone density and/or bone architecture. The primary goal of drug therapy in postmenopausal osteoporosis is to reduce the risk of fractures by increasing bone mass of normal quality.

Alendronate is a nitrogen-containing bisphosphonate which selectively inhibits osteoclast-mediated bone resorption. Like all bisphosphonates, it is chemically related to inorganic pyrophosphate, the endogenous regulator of bone turnover. But while pyrophosphate inhibits both osteoclastic bone resoption and the mineralization of the bone newly formed by osteoblasts, Alendronate specifically inhibits bone resorption without any effect on mineralization at pharmacologically achievable doses. Under therapy, normal bone tissue develops and Alendronate is deposited in the bone-matrix in pharmacologically inactive form.

During treatment with bisphosphonates, the early inhibition of bone resorption can induce a decrease in serum calcium. Vitamin D is required for normal calcium absorption. Thus, adequate vitamin D and calcium intake is desirable for subjects using bisphosphonates. Adequate vitamin D levels become more important when calcium needs are elevated due to net influx of calcium into bone that occurs as a result of Alendronate therapy during osteoporosis treatment.

Vitamin D is a group of fat-soluble prohormones, the two major forms of which are ergocalciferol (vitamin D₂) and cholecalciferol (vitamin D₃). Ergocalciferol is synthesized by plants. Cholecalciferol is synthesized by humans in the skin when it is exposed to UVB rays from sunlight. Although the synthesis of Cholecalciferol occurs naturally in the skin with adequate sunlight exposure, Cholecalciferol is not active and needs to undergo biotransformation in kidney and liver in order to turn into its active form of 1,25-dihydroxycholecalciferol.

The proposed Alendronate/Cholecalciferol combination product of the present invention ensures an adequate level of vitamin D supplementation upon which Alendronate can act.

The chemical name of Alendronate is sodium [4-amino-1-hydroxy-1-(hydroxy-oxido-phosphoryl)-butyl] phosphonic acid trihydrate and its molecular formula is C₄H₁₃NO₇P₂ corresponding to a molecular weight of 249.097. Alendronate sodium is white or almost white crystalline powder, freely soluble in water and practically insoluble in organic solvents.

The chemical name of Cholecalciferol is (3β,5Z,7E)-9,10-secocholesta-5,7,10(19)-trien-3-ol and its molecular formula is C₂₇H₄₄O corresponding to a molecular weight of 384.64. Cholecalciferol is practically insoluble in water and is sensitive to air, heat and light. Therefore, it is presented as Cholecalciferol concentrate (powder form) which is formulated to improve the stability of the active substance. In powder form, Cholecalciferol is dissolved in medium chain triglycerides, embedded in a modified food starch coated matrix based on a combination of gelatin and sucrose to form granules. It also contains butylated hydroxytoluene (BHT) as an antioxidant and magnesium aluminum silicate as an anticaking-agent.

WO 2008/056926 A1 discloses a complex formulation for the prevention or treatment of osteoporosis, comprising: (i) a solid dispersion comprising vitamin D or a derivative thereof and a cyclodextrin, and (ii) a bisphosphonate.

US 6465017 B1 discloses a process for the preparation of tablets comprising as an active ingredient Alendronate comprising the steps of: (a) forming a powder blend of inert pharmaceutical excipients; (b) granulation of the powder with an aqueous solution comprising alendronic acid; and (c) drying the granules to obtain a granulate.

US2010179110 discloses formulations comprising Alendronic acid in the form of a pharmaceutically acceptable salt, Cholecalciferol Vitamin D, microcrystalline cellulose and an anhydrous from of Spray Dried Lactose. Colloidal silicon dioxide is either not required or is required in small amounts. Allegedly those compositions overcome the stickiness typically associated with bisphosphonic acids, however the document is silent with regard to the stability of the disclosed compositions.

EP2201937A1 discloses formulations of vitamin D with alendronic acid in the form of a pharmaceutically acceptable salt, wherein the alendronate salt is dry granulated to form granules in a mixture wherein colloidal silicone dioxide is employed and afterwards mixed with cholecalciferol pellets previously prepared. Allegedly, the pharmaceutical compositions prepared according to this procedure are stable, however the document is silent with regard to the dissolution profile of them.

Although each of the patents above represents an attempt to overcome the stability problems associated with pharmaceutical compositions comprising Alendronate and Cholecalciferol in a single dosage form, there still exists a need for a stable pharmaceutical composition which avoids the problems related to the intrinsic nature of Cholecalciferol.

### SUMMARY OF THE INVENTION

It is an object of the present invention to provide a stable solid pharmaceutical composition for oral administration comprising a nitrogen-containing bisphosphonate such as Alendronate or pharmaceutical acceptable salt, derivative or hydrate thereof and Cholecalciferol as active ingredients in a single dosage form, which overcomes the deficiencies of the prior art.

It is, therefore, an object of the present invention to provide an oral solid dosage formulation comprising Alendronate and Cholecalciferol, which overcomes the tendency of Cholecalciferol to degrade upon exposure to heat, light, air, moisture, oxidizing agents or when exposed to an environment with acidic pH.

It is another object of the present invention to provide a stable combination of Alendronate and Cholecalciferol in a single dosage form so as to increase patient compliance to the treatment. Further object of the present invention is to provide a pharmaceutical composition in the form of a tablet comprising a combination of Alendronate or pharmaceutical acceptable salt, derivative or hydrate thereof and Cholecalciferol as active ingredients, which is bioavailable and has acceptable pharmacotechnical properties.

A major object of the present invention is the selection of the optimal combination of pharmaceutical acceptable excipients in order to achieve the appropriate dissolution profile and stability for the finished dosage form. Said dosage form affords predictable and reproducible drug release rates. Dosage forms with improved properties are advantageous for use in a method of treatment of a patient.

A further approach of the present invention is to provide a method for the preparation of a stable combined solid dosage formulation for oral administration containing Alendronate and Cholecalciferol that overcomes degradation of Cholecalciferol resulting in longer shelf-life of the product.

In accordance with the above objects of the present invention a pharmaceutical composition for oral administration is provided comprising in a single dosage form a combination of Alendronate or pharmaceutical acceptable salt, derivative or hydrate thereof and Cholecalciferol as active ingredients which is free of colloidal silicon dioxide in order to inhibit degradation of Cholecalciferol.

According to another embodiment of the present invention, a process for the preparation of a stable, solid dosage form for oral administration, comprising a nitrogen-containing bisphosphonate such as Alendronate or pharmaceutical acceptable salt, derivative or hydrate thereof and Cholecalciferol, wherein the composition is free of colloidal silicon dioxide in order to inhibit degradation of Cholecalciferol, is provided, which comprises the following steps:
- Mixing of Alendronate sodium with the excipients of internal phase;
- Dry granulation (slugging or roller compaction) of the blend and then sieving through proper sieve;
- Mixing of Cholecalciferol with the granules;
- Lubrication of the mixture with magnesium stearate;
- Compression of the bulk mixture into tablets.

Other objects and advantages of the present invention will become apparent to those skilled in the art in view of the following detailed description.

### DETAILED DESCRIPTION OF THE INVENTION

For the purposes of the present invention, a pharmaceutical composition comprising an active ingredient is considered to be "stable" if said ingredient degrades less or more slowly than it does on its own and/or in known pharmaceutical compositions.

In the present invention Alendronate or pharmaceutical acceptable salt, derivative or hydrate thereof and Cholecalciferol are combined in a single dosage form to achieve desired effect by providing an effective formulation of these two active ingredients.

Patients usually skip taking their medications for reasons of busy life, different time schedules of used drugs, communication problems with the doctor, side effects, cognitive disorders, financial problems and complicated treatment regimens.

A complicated dose regime, especially multiple drug use in several times a day is the most significant factor to prevent the patient's compliance to treatment. The patient compliance and therefore the success of treatment will be higher if the treatment regimen is much simpler. Consequently, the combination of active ingredients in a single dosage form simplifies treatment and thus increases patient's compliance to treatment.

Cholecalciferol is sensitive to air, heat and light. It undergoes degradation reactions at elevated temperatures and high humidity reactions. It is well known that during the development of any pharmaceutical dosage form the impurities should be kept at very low limits not only because they might be harmful for the humans but also because the impurities diminish the potency of pharmaceutical compositions during storage.

Given that Cholecalciferol has the potential to degrade, development focus was targeted at validating a robust, accurate process that would minimize any potential for degradation. The objective of the present invention is to produce a stable drug product with long term stability and therefore ensure the quality of the drug product throughout shelf life.

The most widely used process of agglomeration in pharmaceutical industry is wet granulation. Wet granulation process involves wet massing of the powder blend with a granulating liquid, wet sizing and drying. The greatest disadvantage of wet granulation is its cost. It is an expensive process because of labor, time, equipment, energy and space requirements. Stability may also be major concern for moisture sensitive and thermo labile drugs. Given this, wet granulation is not suitable for preparing tablets comprising Cholecalciferol due to the fact that Cholecalciferol is very sensitive to heat and moisture.

Direct compression is a popular choice because it provides the shortest, most effective and least complex way to produce tablets. The active ingredients are simply blended with the excipients and the lubricant, followed by compression, which makes the product easy to process. No additional processing steps are required. Moisture- or heat-sensitive ingredients, which would be contraindicated in wet granulation, can also be used in this type of process. However, it does require a very critical selection of excipients in comparison to granulation processes because the raw materials must demonstrate good flowability and compressibility for successful operation.

Dry granulation methods are suitable for active ingredients that are sensitive to solvents, or labile to moisture and elevated temperatures. They can also be used for substances possessing poor flowabilty and compaction behaviour. The main advantage of dry granulation is that it uses less equipment and space. It eliminates the need for binder solution, heavy mixing equipment and the costly and time consuming drying step required for wet granulation. There are two types of widely used dry granulation methods: slugging and roller compaction.

Granulation by slugging is the process of compressing dry powder of tablet formulation with tablet press having die cavity large enough in diameter to fill quickly. Once slugs are produced they are reduced to appropriate granule size for final compression by screening and milling.

The roller compactor forces fine powders between two counter rotating rolls and presses the raw materials into a solid compact or sheet, so called flakes. Finally flakes are then reduced in size to the desired grain size.

The excipients used in the present invention were selected with the perspective to enhance dissolution properties and stability of drug substances in the finished dosage form and thus were subjected to compatibility study with both active ingredients, i.e. Alendronate and Cholecalciferol.

It has been surprisingly found that a stable solid pharmaceutical composition for oral administration comprising Alendronate or pharmaceutical acceptable salt, derivative or hydrate thereof and Cholecalciferol in a single dosage form is provided when the composition is free of colloidal silicon dioxide and is produced by dry granulation process. More specifically, the object of the present invention is achieved when Cholecalciferol is in external phase and is mixed with internal phase after slugging or roller compaction of the blend of internal phase.

The "external phase" sits in the composition but outside the internal phase, which may be a granule comprising for example pharmaceutically acceptable excipients or a mixture of pharmaceutically acceptable excipients and active ingredients.

Colloidal silicon dioxide is hygroscopic; when it comes in contact with moisture it acts as humectant adsorbing a significant amount of water. Thus, it was proved to have an impact on Cholecalciferol stability.

Common diluents such as lactose, mannitol, starch and microcrystalline cellulose are comprised in formulations of the present invention in order to enhance flowability and compaction properties of the powder blend.

Croscarmellose sodium as disintegrant provides the necessary force to rapture and eventually disintegrate the tablets. Specifically, due to its large swelling capacity in aqueous solution it enhances the force needed to push particles apart within tablet pores exerted by the water, resulting in rapid tablet disintegration.

At least a lubricant such as magnesium stearate is incorporated into the formulations of the present invention to prevent the powder from adhering to tablet punches during the compression procedure.

In order to obtain the desired therapeutic effect an amount of 70 mg of Alendronic acid and an amount of Cholecalciferol in the range of 2800-5600 IU, most preferably 2800 IU and 5600 IU of Cholecalciferol are comprised in the formulations of the present invention.

The following examples illustrate preferred embodiments in accordance with the present invention without limiting the scope or spirit of the invention.

### EXAMPLES

### Comparative Example 1:

**Table 1: Composition 1 of example 1**

| **Composition 1** | mg |
|---|---|
| **Internal Phase** | |
| Alendronic acid | 70.00 |
| Alendronate Na.3H₂O | 91.37 |
| Cholecalciferol | 56.00 |
| Lactose anhydrous | 51.24 |
| Microcrystalline cellulose | 123.39 |
| Croscarmellose sodium | 17.50 |
| Colloidal silicon dioxide | 7.00 |

| **External Phase** | |
|---|---|
| Magnesium Stearate | 3.50 |
| **Total weight of tablet** | 350.00 |

Composition 1 was prepared according to the following manufacturing process:
- Mixing of Alendronate sodium with Cholecalciferol and excipients of internal phase;
- Lubrication of the mixture with magnesium stearate;
- Compression of the bulk mixture into tablets.

Tablets of Composition 1 were loaded to stability chambers. Stability data after 3 months under normal, intermediate and accelerated conditions are presented in Table 2 below.

**Table 2: Stability results of Composition 1 after 3 months storage**

| | **3 MONTHS** | | |
|---|---|---|---|
| **SPECIFICATION** | **25°C/ 60% RH** | **30°C/65%RH** | **40°C/75%RH** |
| **Total impurities** | 2,58% | 3,79% | 10,74% |

Taking into account the above stability data, it is clear that Composition 1 is not stable since a high percentage of degradation products of Cholecalciferol are formed after storage under normal, intermediate and accelerated conditions.

In order to study the influence of each excipient used in Composition 1 in chemical degradation of Cholecalciferol, the following formulations (Table 3) were prepared in which one excipient at a time was excluded from the formulation and placed under stressed conditions (Table 4).

**Table 3: Compositions 1A to 1E**

| **Composition** | 1A | 1B | 1C | 1D | 1E |
|---|---|---|---|---|---|
| | mg | | | | |
| **Internal Phase** | | | | | |
| Alendronic acid | 70,00 | 70,00 | 70,00 | 70,00 | 70,00 |
| Alendronate sodium trihydrate | 91,37 | 91,37 | 91,37 | 91,37 | 91,37 |
| Cholecalciferol | 56,00 | 56,00 | 56,00 | 56,00 | 56,00 |
| Lactose anhydrous | - | 51,24 | 51,24 | 51,24 | 51,24 |
| Microcrystalline cellulose | 123,39 | - | 123,39 | 123,39 | 123,39 |
| Croscarmellose sodium | 17,50 | 17,50 | - | 17,50 | 17,50 |
| Colloidal silicon dioxide | 7,00 | 7,00 | 7,00 | - | 7,00 |

| **External Phase** | | | | | |
|---|---|---|---|---|---|
| Magnesium Stearate | 3,50 | 3,50 | 3,50 | 3,50 | - |
| **Total weight of tablet** | 298,76 | 226,61 | 332,50 | 343,00 | 346,50 |

**Table 4: Stability results of Compositions 1A to 1E under stressed conditions**

| **SPECIFICATION** | **65°C 15 days** | | | | |
|---|---|---|---|---|---|
| **Compositions** | **1A** | **1B** | **1C** | **1D** | **1E** |
| **Total Impurities** | 5,57% | 3,54% | 3,98% | 0,54% | 3,58% |

The results above show that Composition 1D without colloidal silicon dioxide is stable whereas the rest Compositions 1A-C and 1E which contain colloidal silicon dioxide have a high percentage of impurities most of which are degradation products of Cholecalciferol.

### Comparative Example 2

**Table 5: Compositions 2 & 3 of example 2**

| **Compositions** | 2 | 3 |
|---|---|---|
| | mg | |
| **Internal Phase** | | |
| Alendronic acid | 70.00 | 70.00 |
| Alendronate Na.3H₂O | 91.37 | 91.37 |
| Cholecalciferol | 56.00 | - |
| Lactose anhydrous | 65.25 | 65.25 |
| Microcrystalline cellulose | 122.50 | 122.50 |
| Croscarmellose sodium | 10.50 | 10.50 |
| Colloidal silicon dioxide | 0.88 | 0.88 |
| Magnesium Stearate | - | 2.45 |

| **External Phase** | | |
|---|---|---|
| Cholecalciferol | - | 56.00 |
| Magnesium Stearate | 3.50 | 1.05 |
| **Total weight of tablet** | 350.00 | 350.00 |

Thus, in order to improve chemical stability of Cholecalciferol, Compositions 2 and 3 were prepared by reducing significantly the amount of colloidal silicon dioxide. In addition, the amount of croscarmellose sodium was reduced in order to adjust the disintegration time which was shorter than the desirable.

Composition 2 was prepared with the same manufacturing process as Composition 1.

Tablets of Composition 2 were loaded to stability chambers. Stability data after 3 months under normal, intermediate and accelerated conditions are presented in Table 6 below.

**Table 6: Stability results of Composition 2 after 3 months storage**

| | **3 MONTHS** | | |
|---|---|---|---|
| **SPECIFICATION** | **25°C/ 60% RH** | **30°C/65%RH** | **40°C/75%RH** |
| **Total impurities** | 1,40% | 1,74% | 2,19% |

Composition 3 was prepared with the following dry granulation process. In order to protect Cholecalciferol from extended processing time and exposure in the air, it was mixed with the granules after slugging.
- Mixing of Alendronate sodium with excipients of internal phase;
- Slugging of the blend and then sieving through 1mm sieve;
- Mixing of Cholecalciferol with the granules;
- Lubrication of the mixture with the rest amount of magnesium stearate;
- Compression of the bulk mixture into tablets.

Tablets of Composition 3 were loaded to stability chambers. Stability data after 3 months under normal, intermediate and accelerated conditions are presented in Table 7 below.

**Table 7: Stability results of Composition 3 after 3 months storage**

| | **3 MONTHS** | | |
|---|---|---|---|
| **SPECIFICATION** | **25°C/60% RH** | **30°C/ 65%RH** | **40°C/ 75%RH** |
| **Total impurities** | 1,26% | 1,64% | 1,96% |

Stability results of Compositions 2 and 3 show that the reduction of the amount of colloidal silicon dioxide significantly reduced the degradation of Cholecalciferol. Moreover, Composition 3 that was prepared with dry granulation process instead of direct compression used for the preparation of Composition 2 gave better stability results, i.e. lower percentage of impurities.

### Example 3:

**Table 8: Composition4 to 7 of example 3**

| **Composition** | 4 | 5 | 6 | 7 |
|---|---|---|---|---|
| | mg | | | |
| **Internal Phase** | | | | |
| Alendronic acid | 70.00 | 70.00 | 70.00 | 70.00 |
| Alendronate Na.3H₂O | 91.37 | 91.37 | 91.37 | 91.37 |
| Cholecalciferol | 56.00 | - | - | - |
| Lactose anhydrous | 59.13 | 59.13 | - | 59.13 |
| Microcrystalline cellulose | 122.50 | 122.50 | 122.50 | - |
| Mannitol | - | - | 59.13 | - |
| Starch maize | - | - | - | 122.50 |
| Croscarmellose sodium | 17.50 | 17.50 | 17.50 | 17.50 |
| Magnesium Stearate | - | 2.45 | 2.45 | 2.45 |

| **External Phase** | | | | |
|---|---|---|---|---|
| Cholecalciferol | - | 56.00 | 56.00 | 56.00 |
| Magnesium Stearate | 3.50 | 1.05 | 1.05 | 1.05 |
| **Total weight of tablet** | 350.00 | 350.00 | 350.00 | 350.00 |

However, further improvement of chemical stability of Cholecalciferol was considered necessary; thus, Compositions 4 to 7 without colloidal silicon dioxide were prepared and tested for their chemical stability.

Composition 4 was prepared with the same manufacturing process as Composition 1.

Tablets of Composition 4 were loaded to stability chambers. Stability data after 3 months under normal, intermediate and accelerated conditions are presented in Table 9 below.

**Table 9: Stability results of Composition 4 after 3 months storage**

| | **3 MONTHS** | | |
|---|---|---|---|
| **SPECIFICATION** | **25°C/ 60% RH** | **30°C/65%RH** | **40°C/75%RH** |
| **Total impurities** | 1,11% | 1,26% | 1,68% |

Compositions 5 to 7 were prepared with the same dry granulation process as Composition 3. Different combinations of diluents were also tested.

Tablets of Compositions 5 to 7 were loaded to stability chambers. Stability data after 3 months under normal, intermediate and accelerated conditions are presented in Table 10 below.

**Table 10: Stability results of Compositions 5 to 7 after 3 months storage**

| | **Composition 5** | | |
|---|---|---|---|
| **SPECIFICATION** | **25°C/ 60% RH** | **30°C/65%RH** | **40°C/75%RH** |
| **Total impurities** | 0,91% | 1,01% | 1,16% |

| | **Composition 6** | | |
|---|---|---|---|
| **SPECIFICATION** | **25°C/ 60% RH** | **30°C/65%RH** | **40°C/75%RH** |
| **Total impurities** | 0,87% | 1,14% | 1,75% |

| | **Composition 7** | | |
|---|---|---|---|
| **SPECIFICATION** | **25°C/ 60% RH** | **30°C/65%RH** | **40°C/75%RH** |
| **Total impurities** | 0,90% | 1,18% | 1,26% |

The results presented in Table 10 confirm that formulations that do not contain colloidal silicon dioxide are stable. Moreover, Compositions 5 to 7 that were prepared with dry granulation process and Cholecalciferol was mixed with the granules after slugging were more stable than Composition 4 that did not contain colloidal silicon dioxide but it was prepared with direct compression process and Cholecalciferol was simply mixed with Alendronate sodium and all excipients of internal phase.

The dissolution rate of Alendronate in Compositions 5 to 7 was studied by using USP II apparatus (paddles) at 50 rpm and 900 ml deaerated water. (Table 11)

**Table 11: Dissolution results of Alendronate**

| | **% Alendronate release in H₂O, 50 rpm, 900 ml** | | |
|---|---|---|---|
| **Time (min)** | **Composition 5** | **Composition 6** | **Composition 7** |
| 10 | 87,04 | 81,15 | 83,87 |
| 15 | 97,13 | 97,67 | 98,10 |
| 20 | 97,11 | 102,21 | 101,82 |
| 30 | 99,16 | 102,47 | 103,67 |
| 45 | 100,14 | 103,93 | 104,33 |

The dissolution rate of Cholecalciferol in Compositions 5 to 7 was studied by using USP II apparatus (paddles) at 75 rpm and 500 ml SLS.0.3% (Table 12)

**Table 12: Dissolution results of Cholecalciferol**

| | **% Cholecalciferol release in 0.3% SLS, 75 rpm, 500 ml** | | |
|---|---|---|---|
| **Time (min)** | **Composition 5** | **Composition 6** | **Composition 7** |
| 10 | 93,60 | 88,91 | 83,65 |
| 15 | 95,17 | 91,12 | 88,14 |
| 20 | 98,54 | 92,14 | 92,58 |
| 30 | 99,58 | 93,59 | 95,24 |
| 45 | 101,36 | 96,38 | 96,86 |

The dissolution profile given from the dissolution test of tablets of Composition 5 was the desirable.

The tablets of Compositions 5 to 7 were also tested for their physical characteristics, hardness and disintegration time. (Table 13).

**Table 13: Results of Compositions 5 to 7**

| **RESULTS** | | | |
|---|---|---|---|
| **Physical properties** | **Composition 5** | **Composition 6** | **Composition 7** |
| **Hardness** | 197 N | 183 N | 189 N |
| **Disintegration** | 1'55" - 2'10" | 1'40"-1'55" | 1'55"-2'20" |

The results of Table 13 above show that the tablets of Composition 5 had the desired physicochemical characteristics. The preferred compositions according to the present invention are illustrated in Table 14 below:

**Table 14: Preferred compositions of the present invention**

| **Ingredients** | **mg** | |
|---|---|---|
| **Internal Phase** | | |
| Alendronic acid | 70.00 | 70.00 |
| Alendronate sodium trihydrate | 91.37 | 91.37 |
| Lactose anhydrous | 59.13 | 59.13 |
| Microcrystalline cellulose | 122.50 | 122.50 |
| Croscarmellose sodium | 17.50 | 17.50 |
| Magnesium Stearate | 2.45 | 2.45 |

| **External phase** | | |
|---|---|---|
| Cholecalciferol | 56.00 | 28.00 |
| Magnesium Stearate | 1.05 | 1.05 |
| **Total weight of tablet** | 350.00 | 322.00 |

The preferred compositions of the present invention were prepared according to the following manufacturing process:
- Blending Alendronate sodium with the excipients of internal phase;
- Dry granulation, either slugging or roller compaction, of the blend;
- Sieving through proper sieve;
- Mixing Cholecalciferol with the dry granular mass until uniform mixture is obtained;
- Lubrication of the mixture with the rest amount of magnesium stearate;
- Compression of the resulted mixture into a tablet dosage form.

While the present invention has been described with respect to the particular embodiments, it will be apparent to those skilled in the art that various changes and modifications may be made in the invention without departing from the spirit and scope thereof, as defined in the appended claims.

## Claims

1. A pharmaceutical composition for oral administration comprising in a single dosage form a combination of a therapeutically effective quantity of Alendronate or pharmaceutical acceptable salt, derivative or hydrate thereof and Cholecalciferol, wherein the composition is produced by dry granulation process and comprises an internal phase comprising Alendronate or pharmaceutical acceptable salt, derivative or hydrate thereof and an external phase comprising Cholecalciferol, wherein the composition further comprises croscarmellose sodium as disintegrant and is free of colloidal silicon dioxide.

2. The pharmaceutical composition according to claim 1, further comprising at least one pharmaceutically acceptable excipient selected from diluents and lubricants.

3. The pharmaceutical composition according to claim 2, wherein the diluent is lactose and microcrystalline cellulose.

4. The pharmaceutical composition according to claim 2, wherein the lubricant is magnesium stearate.

5. A process for the preparation of a stable pharmaceutical composition for oral administration comprising in a single dosage form a combination of therapeutically effective quantity of Alendronate or pharmaceutical acceptable salt, derivative or hydrate thereof and Cholecalciferol, wherein the composition is free of colloidal silicon dioxide and Cholecalciferol is in external phase in order to avoid Cholecalciferol degradation, which comprises the steps of:
- Blending Alendronate sodium with lactose, microcrystalline cellulose, croscarmellose sodium and an amount of magnesium stearate;
- Dry granulation, either slugging or roller compaction, of the blend obtained from previous step;
- Sieving through proper sieve;
- Mixing Cholecalciferol with the dry granular mass until uniform mixture is obtained;
- Lubrication of the mixture with the remaining amount of magnesium stearate;
- Compression of the resulted mixture into a tablet dosage form.

## Patentansprüche

1. Pharmazeutische Zusammensetzung zur oralen Verabreichung, umfassend in einer Einzeldosisform eine Kombination einer therapeutisch wirksamen Menge von Alendronat oder eines pharmazeutisch verträglichen Salzes, Derivats oder Hydrats davon und Cholecalciferol, wobei die Zusammensetzung durch ein Trockengranulationsverfahren hergestellt wird und eine innere Phase umfasst Alendronat oder ein pharmazeutisch verträgliches Salz, Derivat oder Hydrat davon und eine externe Phase, umfassend Cholecalciferol, wobei die Zusammensetzung ferner Croscarmellose-Natrium als Sprengmittel umfasst und frei von kolloidalem Siliciumdioxid ist.

2. Pharmazeutische Zusammensetzung nach Anspruch 1, ferner umfassend mindestens einen pharmazeutisch verträglichen Hilfsstoff, ausgewählt aus Verdünnungsmitteln und Schmiermitteln.

3. Pharmazeutische Zusammensetzung nach Anspruch 2, wobei das Verdünnungsmittel Lactose und mikrokristalline Cellulose ist.

4. Pharmazeutische Zusammensetzung nach Anspruch 2, wobei das Schmiermittel Magnesiumstearat ist.

5. Verfahren zur Herstellung einer stabilen pharmazeutischen Zusammensetzung zur oralen Verabreichung, umfassend in einer Einzeldosisform eine Kombination aus einer therapeutisch wirksamen Menge Alendronat oder einem pharmazeutisch verträglichen Salz, Derivat oder Hydrat davon und Cholecalciferol, wobei die Zusammensetzung frei von kolloidalem Siliciumdioxid ist und Cholecalciferol befindet sich in einer externen Phase, um einen Abbau von Cholecalciferol zu vermeiden, der die folgenden Schritte umfasst:
- Mischen von Alendronat-Natrium mit Lactose, mikrokristalliner Cellulose, Croscarmellose-Natrium und einer Menge Magnesiumstearat;
- Trockengranulation der Mischung, die aus dem vorherigen Schritt erhalten wurde, entweder durch Schlagen oder durch Walzenverdichtung;
- Sieben durch richtiges Sieb;
- Mischen von Cholecalciferol mit der trockenen körnigen Masse, bis eine gleichmäßige Mischung erhalten wird;
- Schmierung der Mischung mit der verbleibenden Menge Magnesiumstearat;
- Komprimierung der resultierenden Mischung in eine Tabletten-Dosierungsform

## Revendications

1. Une composition pharmaceutique pour administration orale comprenant, sous forme posologique unique, une combinaison d'une quantité thérapeutiquement efficace d'Alendronate ou un sel, un dérivé ou un hydrate pharmaceutiquement acceptable de celui-ci et de Cholécalciférol, dans laquelle la composition est produite par granulation sèche et comprend une phase interne comprenant l'Alendronate ou un sel, un dérivé ou un hydrate pharmaceutiquement acceptable de celui-ci et une phase externe comprenant le Cholécalciférol, et ladite composition comprend en outre de la croscarmellose sodique comme désintégrant et est exempte de dioxide de silicium colloïdal.

2. La composition pharmaceutique selon la revendication 1, comprenant en outre au moins un excipient pharmaceutiquement acceptable sélectionné parmi des diluants et des lubrifiants.

3. La composition pharmaceutique selon la revendication 2, dans laquelle le diluant est le lactose et la cellulose microcristalline.

4. La composition pharmaceutique selon la revendication 2, dans laquelle le lubrifiant est le stéarate de magnésium.

5. Un procédé pour la préparation d'une composition pharmaceutique stable pour administration orale comprenant sous forme posologique unique une combinaison d'une quantité thérapeutiquement efficace d'Alendronate ou un sel, un dérivé ou un hydrate pharmaceutiquement acceptable de celui-ci et de Cholécalciférol, ladite composition est exempte de dioxide de silicium colloïdal et le Cholécalciférol est dans la phase externe afin d'éviter la dégradation du Cholécalciférol, qui comprend les étapes suivantes :
- Mélanger l'Alendronate sodique avec le lactose, la cellulose microcristalline, la croscarmellose sodique et une quantité de stéarate de magnésium ;
- Granulation sèche, soit par tassement soit par compactage au rouleau, du mélange obtenu à l'étape précédente;
- Passer à travers d'un tamis approprié ;
- Mélanger le Cholécalciférol avec la masse sèche granulaire jusqu'à l'obtention d'un mélange uniforme ;
- Lubrifier le mélange avec la quantité restante de stéarate de magnésium ;
- Compression du mélange résultant sous forme posologique de comprimé.
